# EUROPEAN PATENT SPECIFICATION

(11) **EP 3 554 407 B1**
(45) Date of publication and mention of the grant of the patent: **15.04.2020**
(21) Application number: 17818480.0
(22) Date of filing: 08.12.2017
(51) Int. Cl.: A61B 18/20, A61B 18/00

(54) **A HAIR CUTTING DEVICE AND A METHOD OF OPERATING A HAIR CUTTING DEVICE**
HAARSCHNEIDEVORRICHTUNG UND VERFAHREN ZUM BETRIEB EINER HAARSCHNEIDEVORRICHTUNG
DISPOSITIF DE COUPE DE CHEVEUX ET PROCÉDÉ DE FONCTIONNEMENT D'UN DISPOSITIF DE COUPE DE CHEVEUX

(30) Priority: 13.12.2016 EP 16203717
(43) Date of publication of application: 23.10.2019
(73) Proprietor: Koninklijke Philips N.V., 5656 AG Eindhoven (NL)
(72) Inventor: BOAMFA, Marius, Iosif, 5656 AE Eindhoven (NL); THUMMA, Kiran, Kumar, 5656 AE Eindhoven (NL); MOESKOPS, Bastiaan, Wilhelmus, Maria, 5656 AE Eindhoven (NL); JOHNSON, Mark, Thomas, 5656 AE Eindhoven (NL); VERHAGEN, Rieko, 5656 AE Eindhoven (NL)
(74) Representative: Uittenbroek, Arie Leendert
(86) International application number: PCT/EP2017/081946
(87) International publication number: WO 2018/108718

(56) References cited:
- WO-A1-2014/143670
- US-A1- 2008 244 912
- US-A1- 2008 306 471
- US-A1- 2011 015 621
- US-A1- 2012 123 444

## Description

### FIELD OF THE INVENTION

The invention relates to a hair cutting device for cutting (e.g. shaving) hair on a body of a subject, and in particular relates to a hair cutting device that uses light to cut or shave hair and a method of operating a hair cutting device.

### BACKGROUND OF THE INVENTION

Shaving devices for cutting or shaving hair on a body of a subject typically make use of one or more blades that cut hairs as the blade is moved across the skin of the subject. The blades can be static within the device, for example as in a wet razor, whereas in other types of devices, for example electric shavers, one or more blade elements can be actuated (e.g. rotated or oscillated) in order to produce a cutting action.

However, an alternative type of shaving device has been proposed in WO 2014/143670 that makes use of laser light. In particular a laser light source is provided that is configured to generate laser light having a wavelength selected to target a predetermined chromophore to effectively cut a hair shaft. A fiber optic is located on a shaving portion of the device that is positioned to receive the laser light from the laser light source at a proximal end, conduct the laser light from the proximal end toward a distal end, and emit the light out of a cutting region of the fiber optic and toward hair when the cutting region is brought in contact with the hair.

To achieve good shaving effectiveness, the cutting element of the shaving device (i.e. the fiber optic in the case of the device in WO 2014/143670) needs to be brought very close to the skin and the laser light needs to have sufficient power to cut the hair through melting.

In particular, high intensity laser light is required in the fiber optic to facilitate the initiation of hair cutting. However, the high laser intensity results in high temperatures on the region of the fiber optic where hair cutting (melting) occurs, which is seen experimentally to reach a temperature close to or higher than the glass transition temperature, Tg, of the fiber (in the case of fibers formed from a glass or glass-like material). This is especially the case when low wavelength light (around 450nm) is used for the cutting, as this is strongly absorbed by (darker) hair and as such concentrates the build-up of heat at the position close to the fiber optic.

Repeated heating of the fiber to such high temperatures and subsequent cooling makes the fiber brittle, which in the long term compromises the durability of the fiber.

Therefore there is a need for a hair cutting device and method of operation that improves the durability of a fiber while maintaining hair cutting effectiveness.

### SUMMARY OF THE INVENTION

It has been found that one of the ways to increase the durability of a fiber formed from a glass or glass-like material is to avoid the fiber reaching its glass transition temperature Tg during operation, and this can be achieved by pulsing the laser light during operation. Similarly, for a fiber formed from other types of material, the durability of the fiber can be increased by avoiding the fiber reaching its melting point.

According to a first aspect, there is provided a hair cutting device for cutting hair on a subject, the hair cutting device comprising a light source for generating light at one or more specific wavelengths corresponding to wavelengths absorbed by one or more chromophores in or on hair; a cutting element that comprises an optical waveguide that is coupled at a first end to the light source to receive light, wherein a portion of a sidewall of the optical waveguide forms a cutting face for contacting hair; and a control unit that is coupled to the light source, wherein the control unit is configured to vary the power of the light generated by the light source between first and second power levels during operation such that, on heating of a hair by light coupling from a contact point on the cutting face into the hair, the temperature of the contact point on the cutting face is maintained below a transition temperature for the optical waveguide, wherein the first power level is higher than the second power level.

In some embodiments, the control unit is configured to vary the power of the light generated by the light source between the first and second power levels according to a duty cycle based on a thermal relaxation time, TRT, of the optical waveguide.

In some embodiments, the control unit is configured to control the light source to generate light at the second power level for at least a minimum time period before controlling the light source to generate light at the first power level, wherein the minimum time period is based on a thermal relaxation time, TRT, of the optical waveguide. In these embodiments, the minimum time period can be between 0.001 milliseconds (ms) and 10 ms. In these embodiments, the control unit can also be configured to control the light source to generate light at the first power level after generating light at the second power level for the minimum time period. Alternatively, the control unit can be configured to detect whether hair is in contact with the optical waveguide and to control the light source to generate light at the first power level on detecting that hair is in contact with the optical waveguide, provided that light has been generated at the second power level for at least the minimum time period.

In some embodiments, the control unit is configured to detect whether hair is in contact with the optical waveguide and to control the light source to generate light at the first power level on detecting that hair is in contact with the optical waveguide.

In some embodiments, the control unit is configured to control the light source to generate light at the first power level for up to a maximum time period.

In some embodiments, the optical waveguide is formed from a glass or glass-like material, and wherein the transition temperature is the glass transition temperature for the material. In other embodiments, the optical waveguide is formed from a material having a melting temperature, and the transition temperature is the melting temperature of the material.

According to a second aspect, there is provided a method of operating a hair cutting device to cut hair on a body of a subject, the hair cutting device comprising a cutting element that comprises an optical waveguide, wherein a portion of a sidewall of the optical waveguide forms a cutting face for contacting hair, the method comprising varying the power of light generated by a light source and provided to the optical waveguide between first and second power levels during operation such that, on heating of a hair by light coupling from a contact point on the cutting face into the hair, the temperature of the contact point on the cutting face is maintained below a transition temperature for the optical waveguide, wherein the first power level is higher than the second power level.

In some embodiments, the step of varying comprises varying the power of the light generated by the light source between the first and second power levels according to a duty cycle based on a thermal relaxation time, TRT, of the optical waveguide.

In some embodiments, the step of varying comprises generating light at the second power level for at least a minimum time period before controlling the light source to generate light at the first power level, wherein the minimum time period is based on a thermal relaxation time, TRT, of the optical waveguide. In these embodiments, the minimum time period can be between 0.001 milliseconds (ms) and 10 ms. In these embodiments, the step of varying can also comprise generating light at the first power level after generating light at the second power level for the minimum time period. Alternatively, the method can further comprise the step of detecting whether hair is in contact with the optical waveguide and the step of varying can comprise generating light at the first power level on detecting that hair is in contact with the optical waveguide, provided that light has been generated at the second power level for at least the minimum time period.

In some embodiments, the method can further comprise the step of detecting whether hair is in contact with the optical waveguide and the step of varying can comprise generating light at the first power level on detecting that hair is in contact with the optical waveguide.

In some embodiments, the step of varying comprises generating light at the first power level for up to a maximum time period.

In some embodiments, the optical waveguide is formed from a glass or glass-like material, and wherein the transition temperature is the glass transition temperature for the material. In other embodiments, the optical waveguide is formed from a material having a melting temperature, and the transition temperature is the melting temperature of the material.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a block diagram of a hair cutting device according to an embodiment of the invention;
Fig. 2 is a pair of schematic drawings showing different views of an exemplary hair cutting device according to an embodiment of the invention;
Fig. 3 is a graph illustrating the refractive index of hair;
Fig. 4 is an illustration of an optical fibre cutting element;
Fig. 5 is a flow chart illustrating a method of operating a hair cutting device according to an embodiment; and
Figs. 6, 7 and 8 illustrate three different pulse regimes according to various embodiments.

### DETAILED DESCRIPTION OF THE EMBODIMENTS

As noted above, the present invention provides an improvement in the durability of an optical waveguide (e.g. a fiber optic) in a laser light-based shaving device, for example as described in WO 2014/143670. In particular, a pulsed light regime can be used to prevent the optical waveguide overheating (i.e. reaching the glass transition temperature or melting temperature of the optical waveguide), while still enabling a fast initialisation of hair cutting when hair is in contact with the optical waveguide.

It will be appreciated that the invention is applicable to shaving devices (e.g. razors or electric shavers), and any other type of device that is used to cut hair (e.g. hair clippers), even if those devices do not necessary aim to provide a 'clean shave' (i.e. to remove hair at the level of the skin).

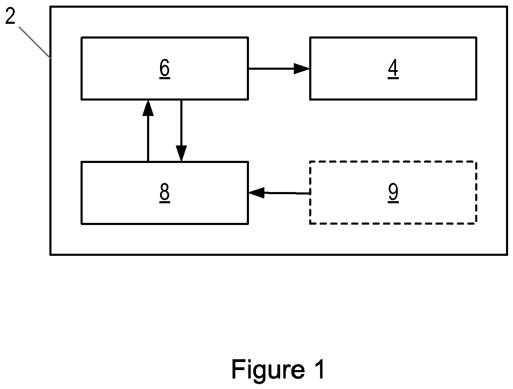

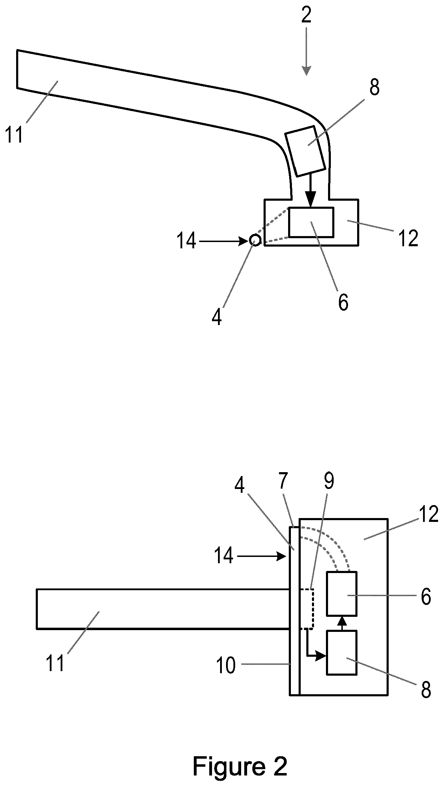

Fig. 1 is a block diagram of a hair cutting device 2 according to an embodiment of the invention. Fig. 2 shows a hair cutting device 2 in the form of a handheld razor according to an exemplary embodiment of the invention. The hair cutting device 2 is for cutting (e.g. shaving) hair on a body of a subject. The subject may be a person or an animal. The hair may be facial hair (i.e. hair on the subject's face), or hair on the subject's head or other part of their body (legs, chest, etc.).

The hair cutting device 2 comprises a cutting element 4 that enables hair to be cut as the hair cutting device 2 is moved over the skin of a subject. The cutting element 4 is an optical waveguide 4 that is arranged on the hair cutting device 2 so that the optical axis of the optical waveguide 4 (i.e. the line along which light typically propagates through the optical waveguide 4) is generally perpendicular to the direction in which the hair cutting device 2 is moved so that hairs contact the side wall of the optical waveguide 4 (the side wall corresponding to the long edge of the optical waveguide 4) as the hair cutting device 2 is moved across the skin of the subject. In some embodiments, the optical waveguide 4 is an optical fibre, although those skilled in the art will be aware of other types of optical waveguide that can be used according to the invention, such as a slab waveguide, a strip waveguide or a photonic crystal waveguide. An optical fibre comprises a core, and in some embodiments also comprises a cladding, which may or may not fully encompass the core (e.g. part of the core may be exposed).

A light source 6 is provided in the hair cutting device 2 that generates laser light at one or more specific wavelengths. The light source 6 is optically coupled to a first end 7 of the optical waveguide 4 so that the laser light generated by the light source 6 is coupled into the optical waveguide 4 (and specifically coupled into an end of the optical waveguide 4 so that the laser light propagates through the optical waveguide 4). As described in more detail below, the power (optical power) of the light generated by the light source 6 can be controlled in accordance with embodiments of the invention. In particular the power of the light can be controlled at least between a first power level and a second power level that is lower than the first power level. In some embodiments, the light source 6 can also be coupled to the end of the optical waveguide 4 opposite the first end 7 so that light is input to the optical waveguide 4 at both ends.

The light source 6 is configured to generate laser light at one or more specific wavelengths that can be used to cut or burn through hair. In particular, each wavelength corresponds to the wavelength of light absorbed by a chromophore that is found in or on hair. As is known, a chromophore is the part of a molecule that provides the molecule with its colour. Thus, the laser light will be absorbed by the chromophore and converted into heat which will melt or burn the hair or otherwise destroy the bonds in the molecules of the hair, and it is this melting or burning that provides the cutting action of the hair cutting device 2.

Suitable chromophores that can be targeted by the laser light generated by the light source 6 include, but are not limited to, melanin, keratin and water. Suitable wavelengths of laser light that can be used include, but are not limited to, wavelengths selected from the range 380 nm (nanometers) to 500 nm and 2500 nm to 3500 nm. Those skilled in the art will be aware of the wavelengths of light that are absorbed by these chromophores, and thus also the specific wavelengths of light that the light source 6 should generate for this purpose, and further details are not provided herein.

In some embodiments the light source 6 can be configured to generate laser light at a plurality of wavelengths (either simultaneously or sequentially), with each wavelength being selected to target a different type of chromophore. This can improve the cutting action of the optical waveguide 4 since multiple types of molecules in the hair may be burnt using the laser light. Alternatively multiple light sources 6 can be provided that each generate laser light at a respective wavelength, and each light source 6 can be coupled to a respective optical waveguide 4 to provide multiple cutting elements in the device 2.

The hair cutting device 2 also comprises a control unit 8 that controls the operation of the hair cutting device 2, and in particular is connected to the light source 6 to control the activation and deactivation of the light source 6, and the power of the light generated by the light source 6. The control unit 8 may activate and deactivate the light source 6 in response to an input from a user of the hair cutting device 2. The control unit 8 can comprise one or more processors, processing units, multi-core processors or modules that are configured or programmed to control the hair cutting device 2. The control unit 8 can also comprise or be associated with a memory or memory module that stores data and computer readable code that is configured to be executed by the control unit 8 to cause the control unit 8 and hair cutting device 2 to operate according to the embodiments described herein.

In accordance with some embodiments of the invention, the hair cutting device 2 can also comprise a sensor 9 that is for detecting whether hair is in contact with the optical waveguide 4. The sensor 9 can be of any suitable type. For example, the sensor 9 can measure the force or pressure acting on the optical waveguide 4 in the normal direction of movement of the hair cutting device 2 (e.g. perpendicular to the optical waveguide 4), and in particular measure forces or pressure of a magnitude that might result from contact between the optical waveguide 4 and hair. Another type of sensor 9 can measure the strain in the optical waveguide 4 as it contacts hair. In another example, the sensor 9 can measure the light level in the optical waveguide 4, such as an end of the optical waveguide 4 opposite the end in which light is input by the light source 6. In this case, the sensor 9 can be any suitable type of sensor, for example a photodiode, photoresistor, phototransistor, etc. The light level in the optical waveguide 4 is dependent on the amount of light input into the optical waveguide 4 and the amount of light coupling out of the optical waveguide 4 into hair. The light level in the optical waveguide 4 measured by sensor 9 can be analysed by the control unit 8 to determine whether hair is in contact with the optical waveguide 4.

As noted above, Fig. 2 shows a hair cutting device 2 that is in the form of a handheld wet razor. Fig. 2 shows a side view and a bottom view of the razor 2. The razor 2 comprises a handle 11 for the subject (or other user of the device 2) to hold, and a head portion 12 that includes the cutting element 4 (optical waveguide/fibre). As shown, the optical waveguide 4 is arranged along an edge of the head portion, and a part of the optical waveguide 4 forms (or corresponds to) a cutting face 14. The cutting face 14 is the part of the optical waveguide 4 that is intended to come into contact with hair as the hair cutting device 2 is moved across the skin of the subject. A light source 6, control unit 8 and sensor 9 (in the bottom view only) are shown as being incorporated into the head portion 12 and handle 11 respectively, but it will be appreciated that the positions of these components in the hair cutting device 2 as shown in Fig. 2 is not limiting. Likewise it will be appreciated that the embodiment shown in Fig. 2 is merely an example, and the cutting element 4, light source 6 and control unit 8 can be incorporated or used in place of a conventional blade in any type of hair cutting device 2 that conventionally comprises a blade for physically cutting or slicing hair (whether the blade is static or actuated in order to achieve a cutting action).

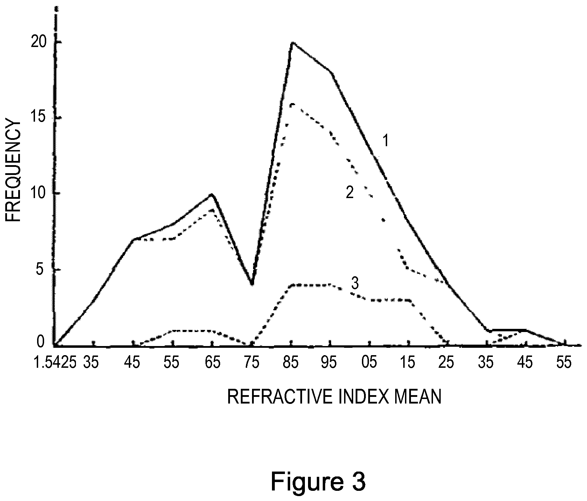

The graph in Fig. 3 illustrates the refractive index of hair, which can be found in a paper by M. D. Greenwell, A. Willner, Paul L. Kirk: Human Hair Studies: III. Refractive Index of Crown Hair, 31 Am. Inst. Crim. L. & Criminology 746 (1940-1941). Curve 1 is a composite line, curve 2 is a line representing the refractive index for Caucasian people, and curve 3 is a line representing the refractive index for non-Caucasian people. Thus, it can be seen that the refractive index of hair is between (approximately) 1.545 and 1.555, although there will be variation between individuals. For example the above paper also recognises that the refractive index of hair can depend on the sex of the subject, e.g. the refractive index of hair on a female is generally higher than the refractive index of hair on a male.

As is known, the optical waveguide 4 acts as a waveguide for the light coupled from the light source 6 through the occurrence of total internal reflection, since the refractive index of air is lower than that of the optical waveguide 4. However, if an object that has a refractive index higher than the optical waveguide 4 is put into contact with the optical waveguide 4, then the total internal reflection is 'frustrated' and light can couple from the optical waveguide 4 into that object. Thus, in order for light to be coupled into a hair from the optical waveguide 4 (to provide the cutting action according to the invention), the optical waveguide 4 must have the same or a lower refractive index than hair at the point at which the hair contacts the optical waveguide 4. Thus, the optical waveguide 4 must have the same or a lower refractive index than hair at least at the cutting face 14 portion of the optical waveguide 4. Preferably the refractive index of the optical waveguide 4 at the cutting face 14 is the same as that of hair since that provides the best coupling of light from the optical waveguide 4 to the hair.

Thus, in some embodiments, the refractive index of the optical waveguide 4 at least at the cutting face 14 is equal to or lower than 1.56. More preferably the refractive index of the optical waveguide 4 at least at the cutting face 14 is equal to or lower than 1.55. Even more preferably, the refractive index of the optical waveguide 14 at least at the cutting face 14 is equal to or lower than 1.54, since this refractive index is below the refractive indices identified in Fig. 3.

In some embodiments, a lower bound for the refractive index of the optical waveguide 4 at the cutting face 14 can be 1.48, 1.51, 1.53 or 1.54.

A range of values from which the refractive index of the optical waveguide 4 is selected can be formed from any combination of the upper and lower refractive index bounds set out in the preceding paragraphs.

The optical waveguide/fibre 4 can be made from any suitable material or combination of materials. For example optical waveguides/fibres can be composed of or comprise a glass or glass-like material, such as silica, fluoride glass, phosphate glass, chalcogenide glass, crown glass (such as BK7), quartz, sapphire, yttrium aluminium garnet, YAG, yttrium aluminosilicates, aluminosilicates, alkaline earth silicates, alkali aluminosilicates, rare-earth doped aluminosilicates, and/or oxyfluorides. It will be appreciated that a glass or glass-like material is a material that has a glass transition temperature at which the material changes from a solid 'glass' state into a viscous state, and which is below the melting temperature of the crystalline state of the material if one exists. In case the material of the optical waveguide 4 is crystalline, then the relevant temperature is the melting temperature rather than a glass transition temperature. In such alternative embodiments, the optical waveguide 4 can be composed of or comprise a non-glass-like material, i.e. a material that does not have a glass transition temperature. Examples of suitable materials include crystalline sapphire and crystalline YAG.

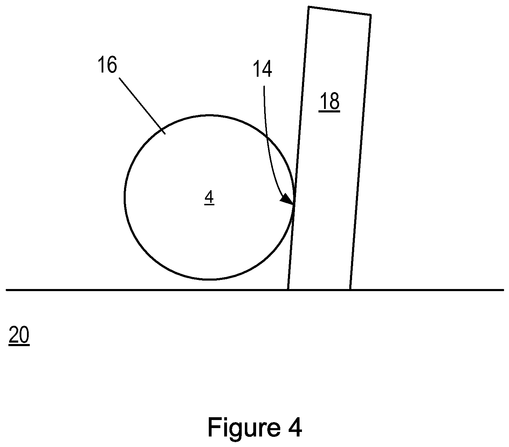

Fig. 4 illustrates an exemplary embodiment of the optical waveguide 4. The optical waveguide 4 is for use in or with a hair cutting device 2, for example as shown in Figs. 1 and 2. In Fig. 4 the optical waveguide 4 is shown side on (i.e. looking down the optical axis of the optical waveguide 4), and no other support element for the optical waveguide 4 is shown.

In Fig. 4, the optical waveguide 4 has a core 16. In this illustrated embodiment, the optical waveguide 4 does not include any cladding around the core 16. However it will be appreciated that in some embodiments the optical waveguide 4 can comprise cladding around the core 16, although preferably no cladding is present along the cutting face 14 (and indeed, in some embodiments the cutting face 14 can correspond to those parts of the optical waveguide 4 where there is no cladding).

The optical waveguide 4 is shown in contact with a hair 18 and close to, but not in contact with, the skin 20. The portion of the side wall of the core 16/optical waveguide 4 that is intended to contact hairs during use forms the cutting face 14. As described above, the refractive index of the core 16 is the same or lower than the refractive index of hair.

The core 16 may have a uniform refractive index (i.e. the same refractive index throughout the core 16), or it may be a graded index fibre, which means that the refractive index decreases with increasing distance from the optical axis.

When the hair cutting device 2 is operated with the light source 6 outputting light at a constant light intensity that is sufficiently high to melt hairs, it has been found that the heating of the hair 18 also heats the part of the optical waveguide 4 in contact with the hair 18, and this heating can increase the temperature of the optical waveguide 4 at that contact point beyond a transition temperature of the optical waveguide 4 (i.e. beyond the glass transition temperature in the case of glass or glass-like optical waveguides 4, or beyond the melting temperature in the case of other optical waveguides 4).

Thus, to avoid or prevent the temperature of the optical waveguide 4 from reaching or exceeding the transition temperature (e.g. glass transition temperature Tg for the optical waveguide 4 (for example around 1200°C for fused silica)), the control unit 8 controls the light source 6 to vary the power of the generated light between first and second power levels during operation (with the first power level being higher than the second power level). The first power level is preferably high enough to initiating a cutting (melting) process, and the second power level can be lower than the first power level and such that the melting/cutting of the hair 18 can continue, without the heating of the hair 18 by the light at the second power level increasing the temperature at the contact point on the cutting face 14 to or beyond the transition temperature of the optical waveguide 4. The first power level can be 150mW or higher, but it will be appreciated that the required power level can depend on one or more factors such as the diameter of the optical waveguide 4, a tapering ratio of the optical waveguide 4 (in the event that the optical waveguide 4 tapers) and/or the coupling medium. The second power level can be 150mW or less. The control unit 8 can control the light source 6 to switch between generating light at the first power level and light at the second power level over time to effect a cutting action of hairs. As well as using the second (lower) power level, the duty cycle of the switching between the first power level and the second power level (i.e. the ratio of the time at the first power level to the time at the second power level) can be selected to ensure that the optical waveguide 4 is not heated to or beyond the transition temperature (e.g. Tg or the melting temperature).

The power levels and the duty cycle of the switching between power levels can be selected based on the properties of the optical waveguide 4, for example the transition temperature and/or the thermal relaxation time of the optical waveguide 4. As is known, the thermal relaxation time is the time required by an object to cool down to a certain proportion of the initial temperature (for example 50% or 66% of the initial temperature). Generally, the shorter the thermal relaxation time (i.e. the quicker the optical waveguide 4 loses heat), the longer (proportionally as part of the duty cycle) the light source 6 can generate light at the first power level, in order to maintain or maximise cutting effectiveness.

Thus, in some embodiments, the power of the generated light is modulated with a pulse width that is proportional to the thermal relaxation time of the optical waveguide 4. That is, the frequency and duty cycle of the switching between the first power level and the second power level can match the thermal relaxation time of the optical waveguide 4. That is, the time between two consecutive high intensity pulses (i.e. pulses at the first power level) should be longer than the thermal relaxation time of the optical waveguide 4. The first power level can be considered as a high intensity pulse which will initiate the cutting process and the second power level can be a lower intensity or even a zero intensity part of the pulse will assist in the 'runaway' process of the hair cutting while keeping the optical waveguide 4 at a relatively low temperature (and in particular compared to using light at a constant intensity or power level).

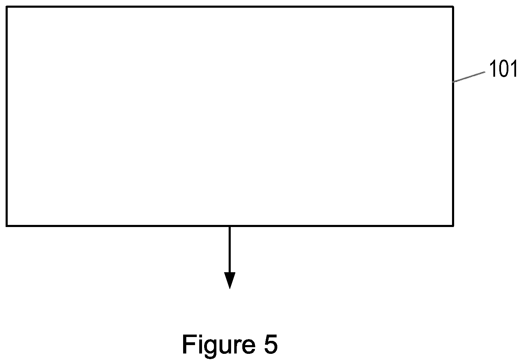

The flow chart in Fig. 5 illustrates a method of operating a hair cutting device 2 to cut hair on a body of a subject (e.g. hair on the head, face, neck, torso, arms or legs). The method comprises, in step 101, varying the power of light generated by a light source 6 between first and second power levels during operation such that, on heating of a hair by light coupling from a contact point on the cutting face 14 into the hair 18, the temperature of the contact point on the cutting face 14 is maintained below a transition temperature for the optical waveguide 4 (i.e. a glass transition temperature in the case of glass or glass-like optical waveguides 4, or a melting temperature in the case of other optical waveguides 4). The first power level is higher than the second power level.

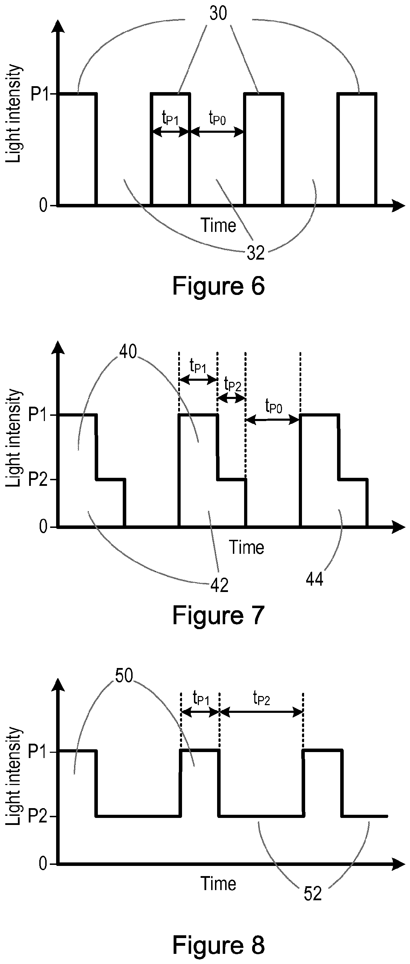

Figs. 6, 7 and 8 illustrate three different pulse regimes according to the invention. Fig. 6 shows a first regime in which the light intensity or power of the generated light is varied between a first power level, PI, and a second power level, which, in this case, is 0. In other words in the regime of Fig. 6, the light source 6 is switched on and off. Fig. 6 shows four high intensity pulses 30 (i.e. pulses at the first power level) and three zero intensity 'gaps' 32. The duration of each high intensity pulse 30 is denoted t_{P1} and the duration of each gap 32 is denoted t_{P0}, and thus the pulse regime has a period of t_{P1} + t_{P0}.

Fig. 7 shows a second regime in which the light intensity or power of the generated light is varied between a first power level, PI, a second power level, P2, and a third power level, which is 0. In particular, the light intensity is switched from the first power level to the second power level and then to 0, before switching back to the first power level and repeating. Fig. 7 shows three high intensity pulses 40 (i.e. pulses at the first power level), followed by respective lower intensity pulses 42 (i.e. pulses at the second power level), and respective zero intensity 'gaps' 44. The duration of each high intensity pulse 40 is denoted t_{P1}, the duration of each lower intensity pulse 42 is denoted t_{P2} and the duration of each gap 44 is denoted t_{P0}, and thus the pulse regime has a period of t_{P1} + t_{P2} + t_{P0}.

Fig. 8 shows a third regime in which the light intensity or power of the generated light is varied between a first power level, P1, and a second (non-zero) power level, P2. Fig. 8 shows three high intensity pulses 50 (i.e. pulses at the first power level) and three lower intensity pulses 52 (i.e. pulses at the second power level). The duration of each high intensity pulse 50 is denoted t_{P1} and the duration of each lower intensity pulse 52 is denoted t_{P2}, and thus the pulse regime has a period of t_{P1} + t_{P2}.

In some embodiments, step 101 comprises varying the power of the light generated by the light source 6 between the first and second power levels according to a duty cycle based on a thermal relaxation time (TRT) of the optical waveguide 4. Thus, the values of t_{P1}, t_{P2} and t_{P0} in Figs. 6, 7 and 8 (or the relationship between these values) can depend on the TRT of the optical waveguide 4. In general, the shorter the TRT (i.e. the quicker the optical waveguide 4 loses heat), the longer the high intensity pulse 30/40/50 can be (or rather the larger the high intensity pulse 30/40/50 can be relative to the pulse 32/42/52 at the second power level).

In some embodiments, step 101 can comprise generating light at the second power level for at least a minimum time period before generating light at the first power level, with the minimum time period being based on the TRT of the optical waveguide 4. Thus, by setting the minimum time period according to the TRT, the optical waveguide 4 can be provided with sufficient cooling time before a further high intensity pulse 30/40/50 is generated and further heating of the optical waveguide 4 occurs. Depending on the construction of the optical waveguide 4, the minimum time period can be between 0.001 milliseconds, ms, and 10 ms. Thus, in some embodiments, t_{P0} in Fig. 6 can be between 0.001 ms, and 10 ms, t_{P2} or t_{P0} (or even t_{P2} + t_{P0}) in Fig. 7 can be between 0.001 ms, and 10 ms, and t_{P2} in Fig. 8 can be between 0.001 ms, and 10 ms.

In some embodiments, since the high intensity pulse 30/40/50 is the pulse that causes the most significant increase in the temperature of the optical waveguide 4 during a cutting/melting operation, the duration of the high intensity pulse 30/40/50 (t_{P1}) is restricted to a maximum duration, based on the magnitude of the first power level and/or properties of the optical waveguide 4, for example the TRT and/or the thermal conductivity. For example, the maximum duration of the high intensity pulse 30/40/50 can be approximately 10 ms at an intensity of 0.5 Watts (W), 40 ms for an intensity of 0.35 W, and 100ms for an intensity of 0.25 W.

Thus, in general the short high intensity (first power level) pulses initiate the cutting process of the hair, but as the pulse width (i.e. t_{P1}) is short, the temperature of the optical waveguide 4 will not reach its transition temperature (e.g. glass transition temperature Tg). After the initiation of the cutting process, the light intensity is reduced as shown in Figs. 6, 7 or 8 to limit the hair temperature during the runaway process of the cutting/melting of hair. It will be appreciated that after the initiation of the hair cutting process, low intensity light can be sufficient to maintain the cutting process.

In some embodiments, to improve the speed with which the cutting process is initiated, the high intensity (first power level) pulse 30/40/50 can be triggered when the optical waveguide 4 contacts hair. Thus, sensor 9 can be used to detect whether the optical waveguide 4 is in contact with hair, and the control unit 8 can control the light source 6 to generate light at the first power level when contact with hair is detected. However, the control unit 8 can only trigger the light source 6 to generate light at the first power level if the light source 6 has been generating light at the second power level for at least a minimum amount of time (e.g. based on the thermal relaxation time of the optical waveguide 4). This ensures that the optical waveguide 4 has enough time to cool down from the previous high intensity pulse before another high intensity pulse occurs. This triggering of the high intensity pulse can also provide improvements to the cutting efficiency since the high intensity pulse will be triggered when hair contacts the optical waveguide 4, maximising the amount of time that the light is at the first power level and can couple into the hair before the power needs to be reduced to the second power level according to the duty cycle.

There is therefore provided an improved hair cutting device and method of operation that improves the durability of an optical waveguide, since the optical waveguide is not heated to its transition temperature, while maintaining hair cutting effectiveness.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage.

Any reference signs in the claims should not be construed as limiting the scope.

## Claims

1. A hair cutting device for cutting hair on a subject, the hair cutting device comprising:
a light source for generating light at one or more specific wavelengths corresponding to wavelengths absorbed by one or more chromophores in or on hair;
a cutting element that comprises an optical waveguide that is coupled at a first end to the light source to receive light, wherein a portion of a sidewall of the optical waveguide forms a cutting face for contacting hair; and
a control unit that is coupled to the light source, wherein the control unit is configured to vary the power of the light generated by the light source between first and second power levels during operation such that, on heating of a hair by light coupling from a contact point on the cutting face into the hair, the temperature of the contact point on the cutting face is maintained below a transition temperature for the optical waveguide, wherein the first power level is higher than the second power level.

2. A hair cutting device as claimed in claim 1, wherein the control unit is configured to vary the power of the light generated by the light source between the first and second power levels according to a duty cycle based on a thermal relaxation time, TRT, of the optical waveguide.

3. A hair cutting device as claimed in claim 1 or 2, wherein the control unit is configured to control the light source to generate light at the second power level for at least a minimum time period before controlling the light source to generate light at the first power level, wherein the minimum time period is based on a thermal relaxation time, TRT, of the optical waveguide.

4. A hair cutting device as claimed in claim 3, wherein the minimum time period is between 0.001 milliseconds, ms, and 10 ms.

5. A hair cutting device as claimed in claim 3 or 4, wherein the control unit is configured to control the light source to generate light at the first power level after generating light at the second power level for the minimum time period.

6. A hair cutting device as claimed in claim 3 or 4, wherein the control unit is configured to detect whether hair is in contact with the optical waveguide and to control the light source to generate light at the first power level on detecting that hair is in contact with the optical waveguide, provided that light has been generated at the second power level for at least the minimum time period.

7. A hair cutting device as claimed in claim 1 or 2, wherein the control unit is configured to detect whether hair is in contact with the optical waveguide and to control the light source to generate light at the first power level on detecting that hair is in contact with the optical waveguide.

8. A hair cutting device as claimed in any of claims 1-7, wherein the control unit is configured to control the light source to generate light at the first power level for up to a maximum time period.

9. A hair cutting device as claimed in any of claims 1-8, wherein the optical waveguide is formed from a glass or glass-like material, and wherein the transition temperature is the glass transition temperature for the material.

10. A method of operating a hair cutting device to cut hair on a body of a subject, the hair cutting device comprising a cutting element that comprises an optical waveguide, wherein a portion of a sidewall of the optical waveguide forms a cutting face for contacting hair, the method comprising:
varying the power of light generated by a light source and provided to the optical waveguide between first and second power levels during operation such that, on heating of a hair by light coupling from a contact point on the cutting face into the hair, the temperature of the contact point on the cutting face is maintained below a transition temperature for the optical waveguide, wherein the first power level is higher than the second power level.

11. A method as claimed in claim 10, wherein the step of varying comprises varying the power of the light generated by the light source between the first and second power levels according to a duty cycle based on a thermal relaxation time, TRT, of the optical waveguide.

12. A method as claimed in claim 10 or 11, wherein the step of varying comprises generating light at the second power level for at least a minimum time period before generating light at the first power level, wherein the minimum time period is based on a thermal relaxation time, TRT, of the optical waveguide.

13. A method as claimed in claim 11, wherein the step of varying comprises generating light at the first power level after generating light at the second power level for the minimum time period.

14. A method as claimed in claim 12, the method further comprising the steps of:
detecting whether hair is in contact with the optical waveguide; and
generating light at the first power level on detecting that hair is in contact with the optical waveguide, provided that light has been generated at the second power level for at least the minimum time period.

15. A method as claimed in claim 10 or 11, the method further comprising the steps of:
detecting whether hair is in contact with the optical waveguide; and
generating light at the first power level on detecting that hair is in contact with the optical waveguide.

## Patentansprüche

1. Eine Haarschneidevorrichtung zum Schneiden von Haaren an einem Probanden, wobei die Haarschneidevorrichtung aus Folgendem besteht:
einer Lichtquelle zur Erzeugung von Licht bei einer oder mehreren spezifischen Wellenlängen, die Wellenlängen entsprechen, die von einem oder mehreren Chromophoren in oder auf Haaren absorbiert werden;
einem Schneidelement, das einen optischen Wellenleiter umfasst, der an einem ersten Ende mit der Lichtquelle gekoppelt ist, um Licht zu empfangen, wobei ein Teil einer Seitenwand des optischen Wellenleiters eine Schneidfläche zum Kontaktieren von Haaren bildet; und
einer Steuereinheit, die mit der Lichtquelle gekoppelt ist, wobei die Steuereinheit dazu konfiguriert ist, die Leistung des von der Lichtquelle erzeugten Lichts während des Betriebs zwischen einer ersten und einer zweiten Leistungsstufe zu variieren, so dass bei Erwärmung eines Haars durch Lichteinkopplung von einem Kontaktpunkt auf der Schneidfläche in das Haar die Temperatur des Kontaktpunkts auf der Schneidfläche unter einer Übergangstemperatur für den Lichtwellenleiter gehalten wird, wobei die erste Leistungsstufe höher als die zweite Leistungsstufe ist.

2. Eine Haarschneidevorrichtung nach Anspruch 1, wobei die Steuereinheit dazu konfiguriert ist, die Leistung des von der Lichtquelle erzeugten Lichts zwischen der ersten und der zweiten Leistungsstufe gemäß einem Arbeitszyklus basierend auf einer thermischen Relaxationszeit (TRZ) des optische Wellenleiters zu variieren.

3. Eine Haarschneidevorrichtung nach Anspruch 1 oder 2, wobei die Steuereinheit dazu konfiguriert ist, die Lichtquelle so zu steuern, dass sie Licht auf der zweiten Leistungsstufe für mindestens eine minimale Zeitdauer erzeugt, bevor sie die Lichtquelle so steuert, dass sie Licht auf der ersten Leistungsstufe erzeugt, wobei die minimale Zeitdauer auf einer thermischen Relaxationszeit (TRZ) des Lichtwellenleiters basiert.

4. Eine Haarschneidevorrichtung nach Anspruch 3, wobei die minimale Zeitdauer zwischen 0,001 Millisekunden (ms) und 10 ms liegt.

5. Eine Haarschneidevorrichtung nach Anspruch 3 oder 4, wobei die Steuereinheit dazu konfiguriert ist, die Lichtquelle so zu steuern, dass sie Licht bei der ersten Leistungsstufe erzeugt, nachdem Licht bei der zweiten Leistungsstufe für den minimalen Zeitraum erzeugt wurde.

6. Eine Haarschneidevorrichtung nach Anspruch 3 oder 4, wobei die Steuereinheit dazu konfiguriert ist, zu erfassen, ob Haare mit dem optischen Wellenleiter in Kontakt sind, und um die Lichtquelle so zu steuern, dass sie Licht bei der ersten Leistungsstufe erzeugt beim Erfassen, dass Haare in Kontakt mit dem optischen Wellenleiter sind, vorausgesetzt, dass Licht für mindestens die minimale Zeitdauer auf der zweiten Leistungsstufe erzeugt wurde.

7. Eine Haarschneidevorrichtung nach Anspruch 1 oder 2, wobei die Steuereinheit dazu konfiguriert ist, um zu erfassen, ob Haare mit dem optischen Wellenleiter in Kontakt stehen, und um die Lichtquelle so zu steuern, dass sie Licht bei der ersten Leistungsstufe erzeugt beim Erfassen, dass Haare in Kontakt mit dem optischen Wellenleiter sind.

8. Eine Haarschneidevorrichtung nach einem der Ansprüche 1 bis 7, wobei die Steuereinheit dazu konfiguriert ist, die Lichtquelle so zu steuern, dass sie Licht bei der ersten Leistungsstufe für einen maximalen Zeitraum erzeugt.

9. Eine Haarschneidevorrichtung nach einem der Ansprüche 1 bis 8, wobei der optische Wellenleiter aus einem Glas oder einem glasartigen Material gebildet ist und wobei die Übergangstemperatur die Glasübergangstemperatur für das Material ist.

10. Verfahren zum Betreiben einer Haarschneidevorrichtung zum Schneiden von Haaren an einem Körper eines Probanden, wobei die Haarschneidevorrichtung ein Schneidelement umfasst, das einen optischen Wellenleiter umfasst, wobei ein Teil einer Seitenwand des optischen Wellenleiters eine Schneidfläche zum Kontaktieren von Haaren bildet; wobei das Verfahren Folgendes umfasst:
Variieren der Leistung von Licht, das von einer Lichtquelle erzeugt und dem Lichtwellenleiter zugeführt wird, zwischen einer ersten und einer zweiten Leistungsstufe während des Betriebs, so dass bei Erwärmung eines Haares durch Lichteinkopplung von einem Kontaktpunkt auf der Schneidfläche in das Haar die Temperatur des Kontaktpunktes auf der Schneidfläche unter einer Übergangstemperatur für den Lichtwellenleiter gehalten wird, wobei die erste Leistungsstufe höher als die zweite Leistungsstufe ist.

11. Ein Verfahren nach Anspruch 10, wobei der Schritt des Variierens das Variieren der Leistung des von der Lichtquelle erzeugten Lichts zwischen dem ersten und dem zweiten Leistungspegel gemäß einem Arbeitszyklus basierend auf einer thermischen Relaxationszeit (TRZ) des optischen Wellenleiters umfasst.

12. Ein Verfahren nach Anspruch 10 oder 11, wobei der Schritt des Variierens die Erzeugung von Licht auf der zweiten Leistungsstufe für mindestens eine minimale Zeitdauer umfasst, bevor Licht auf der ersten Leistungsstufe erzeugt wird, wobei die minimale Zeitdauer auf einer thermischen Relaxationszeit (TRZ) des optischen Wellenleiters basiert.

13. Ein Verfahren nach Anspruch 11, wobei der Schritt des Variierens die Erzeugung von Licht auf der ersten Leistungsstufe nach der Erzeugung von Licht auf der zweiten Leistungsstufe für den minimalen Zeitraum umfasst.

14. Ein Verfahren nach Anspruch 12, wobei das Verfahren ferner die folgenden Schritte umfasst:
Erfassung, ob Haare mit dem optischen Wellenleiter in Kontakt sind; und
Erzeugung von Licht bei der ersten Leistungsstufe beim Erkennen, dass Haare in Kontakt
mit dem optische Wellenleiter sind, vorausgesetzt, dass Licht für mindestens die minimale Zeitdauer mit der zweiten Leistungsstufe erzeugt wurde.

15. Ein Verfahren nach Anspruch 10 oder 11, wobei das Verfahren ferner die folgenden Schritte umfasst:
Erkennung, ob Haare mit dem optischen Wellenleiter in Kontakt sind; und
Erzeugung von Licht bei der ersten Leistungsstufe beim Erkennen, dass Haare in Kontakt
mit dem optischen Wellenleiter sind.

## Revendications

1. Dispositif de coupe de cheveux permettant de couper les cheveux sur un sujet, ledit dispositif de coupe de cheveux comprenant:
une source lumineuse pour générer la lumière à au moins une longueur d'onde spécifique correspondant à des longueurs d'onde absorbées par au moins un chromophore dans ou sur les cheveux;
un élément de coupe, lequel comprend un guide d'ondes optique, lequel est couplé, à une première extrémité, à la source lumineuse pour recevoir la lumière, dans lequel une partie d'une paroi latérale du guide d'ondes optique forme une face de coupe destinée à entrer en contact avec les cheveux; et
une unité de commande, laquelle est couplée à la source lumineuse, dans lequel l'unité de commande est conçue pour faire varier la puissance de la lumière générée par la source lumineuse entre les premier et second niveaux de puissance lors du fonctionnement de telle sorte que, lors du chauffage d'un cheveu par couplage de lumière à partir d'un point de contact sur la face de coupe dans les cheveux, la température du point de contact sur la face de coupe est maintenue inférieure à une température de transition pour le guide d'ondes optique, dans lequel le premier niveau de puissance est supérieur au second niveau de puissance.

2. Dispositif de coupe de cheveux selon la revendication 1, dans lequel l'unité de commande est conçue pour faire varier la puissance de la lumière générée par la source lumineuse entre les premier et second niveaux de puissance en fonction d'un rapport cyclique basé sur un temps de relaxation thermique, TRT, du guide d'ondes optique.

3. Dispositif de coupe de cheveux selon la revendication 1 ou 2, dans lequel l'unité de commande est conçue pour commander la source lumineuse pour générer la lumière au second niveau de puissance pendant au moins une période de temps minimale avant de commander la source lumineuse pour générer la lumière au premier niveau de puissance, dans lequel la période de temps minimale est basée sur un temps de relaxation thermique, TRT, du guide d'ondes optique.

4. Dispositif de coupe de cheveux selon la revendication 3, dans lequel la période de temps minimale est comprise dans la plage entre 0,001 milliseconde, ms, et 10 ms.

5. Dispositif de coupe de cheveux selon la revendication 3 ou 4, dans lequel l'unité de commande est conçue pour commander la source de lumière pour générer la lumière au premier niveau de puissance après avoir généré la lumière au second niveau de puissance pendant la période de temps minimale.

6. Dispositif de coupe de cheveux selon la revendication 3 ou 4, dans lequel l'unité de commande est conçue pour détecter si les cheveux sont en contact avec le guide d'ondes optique et pour commander la source lumineuse pour générer la lumière au premier niveau de puissance lorsqu'il a été détecté que les cheveux sont en contact avec le guide d'ondes optique, à condition que la lumière ait été générée au second niveau de puissance pendant au moins la période de temps minimale.

7. Dispositif de coupe de cheveux selon la revendication 1 ou 2, dans lequel l'unité de commande est conçue pour détecter si les cheveux sont en contact avec le guide d'ondes optique et pour commander la source lumineuse pour générer la lumière au premier niveau de puissance lorsqu'il a été détecté que les cheveux sont en contact avec le guide d'ondes optique.

8. Dispositif de coupe de cheveux selon l'une quelconque des revendications 1 à 7, dans lequel l'unité de commande est conçue pour commander la source lumineuse pour générer la lumière au premier niveau de puissance pendant une période de temps maximale.

9. Dispositif de coupe de cheveux selon l'une quelconque des revendications 1 à 8, dans lequel le guide d'ondes optique est formé d'un verre ou d'une matière semblable au verre, et dans lequel la température de transition est la température de transition vitreuse pour la matière.

10. Procédé de fonctionnement d'un dispositif de coupe de cheveux permettant de couper les cheveux sur le corps d'un sujet, le dispositif de coupe de cheveux comprenant un élément de coupe, lequel comprend un guide d'ondes optique, dans lequel une partie d'une paroi latérale du guide d'ondes optique forme une face de coupe pour entrer en contact avec les cheveux, ledit procédé consistant:
à faire varier la puissance de la lumière générée par une source lumineuse et à fournir au guide d'ondes optique entre les premier et second niveaux de puissance lors du fonctionnement de telle sorte que, lors du chauffage d'un cheveu par couplage de la lumière à partir d'un point de contact sur la face de coupe dans les cheveux, la température du point de contact sur la face de coupe est maintenue inférieure à une température de transition pour le guide d'ondes optique, dans lequel le premier niveau de puissance est supérieur au second niveau de puissance.

11. Procédé selon la revendication 10, dans lequel l'étape de variation comprend la variation de la puissance de la lumière générée par la source lumineuse entre les premier et second niveaux de puissance conformément à un cyclique de service basé sur un temps de relaxation thermique, TRT, du guide d'ondes optique.

12. Procédé selon la revendication 10 ou 11, dans lequel l'étape de variation comprend la génération de la lumière au second niveau de puissance pendant au moins une période de temps minimale avant de générer la lumière au premier niveau de puissance, dans lequel la période de temps minimale est basée sur un temps de relaxation thermique, TRT, du guide d'ondes optique.

13. Procédé selon la revendication 11, dans lequel l'étape de variation comprend la génération de la lumière au premier niveau de puissance après la génération de lumière au second niveau de puissance pendant la période de temps minimale.

14. Procédé selon la revendication 12, ledit procédé comprenant en outre les étapes consistant:
à détecter si les cheveux sont en contact avec le guide d'ondes optique; et à générer la lumière au premier niveau de puissance lorsqu'il a été détecté que les cheveux sont en contact avec le guide d'ondes optique, à condition que la lumière ait été générée au second niveau de puissance pendant au moins la période de temps minimale.

15. Procédé selon la revendication 10 ou 11, ledit procédé comprenant en outre les étapes suivantes:
la détection si les cheveux sont en contact avec le guide d'ondes optique; et la génération de la lumière au premier niveau de puissance lorsqu'il a été détecté que les cheveux sont en contact avec le guide d'ondes optique.
